# EUROPEAN PATENT APPLICATION

(11) **EP 4 431 144 A1**
(43) Date of publication of application: **18.09.2024**
(21) Application number: 22890467.8
(22) Date of filing: 07.11.2022
(51) Int. Cl.: A61M 37/00, A45D 19/00, A45D 29/00, A45D 44/00

(54) **PRINTING DEVICE COUPLABLE TO VARIOUS ASSISTANCE DEVICES AND OPERATION METHOD THEREOF**

(30) Priority: 08.11.2021 KR 20210152412; 04.11.2022 KR 20220145808
(71) Applicant: Prinker Korea Inc., Gyeonngi-do 16426 (KR)
(72) Inventor: LEE, Kyu Suk, Suwon-si Gyeonggi-do 16687 (KR); LEE, Jong In, Suwon-si, Gyeonggi-do 16689 (KR); YUN, Tae Sik, Seoul 06344 (KR)
(74) Representative: Bjerkén Hynell KB
(86) International application number: PCT/KR2022/017319
(87) International publication number: WO 2023/080736

(57) **Abstract**

Provided is a print device including a body forming an appearance, a coupling unit formed in the body to enable physical coupling to an auxiliary device, a recognition unit formed in the body to recognize the auxiliary device and determine whether the coupling unit is coupled to the auxiliary device, and a control unit. When the recognition unit recognizes that the auxiliary device is not coupled to the coupling unit, the control unit sets the auxiliary device to perform a basic function, and when the recognition unit recognizes that the auxiliary device is coupled to the coupling unit, the control unit sets the coupled auxiliary device to perform an individual function changed from the basic function.

## Description

### BACKGROUND

The present disclosure relates to a print device couplable to various auxiliary devices and an operating method of the print device, and more specifically, to a print device that recognizes coupling to each of different auxiliary devices and has different functions according to the recognized auxiliary device and an operating method of the print device.

As people's interest in beauty is continuously increased, various types of cosmetic procedures, which apply color paint to parts of the body, such as tattoos, nail art, and dyeing, are being performed. However, most cosmetic procedures, such as nail art, tattoos, and hair dyeing, require a person with specialized skills to perform the procedure, which causes an issue of lack of accessibility for consumers.

Accordingly, the demand for print technology that may print color paint in the form desired by a user is increasing. The relevant technology is implemented by using a print device, such as a printer that prints color paint, to print paint on a target according to a user's needs. However, since a general print device is manufactured as an integrated device, the general print device has an issue of being bulky and having poor portability. Also, when the output targets, such as skin and hair, are different from each other, an individual print device has to be implemented for each individual.

Therefore, the need for technology emerges in which the print device further includes auxiliary devices, the print device takes charge of most functions, such as, printing, and the auxiliary devices that perform different individual functions may be used by being coupled to the print device.

### SUMMARY

The present disclosure provides a print device configured to recognize coupling to each of different auxiliary devices and to have different functions depending on the recognized auxiliary devices, and an operating method of print device.

Technical objects to be achieved by the present disclosure are not limited to the technical object described above, and other technical objects of the present disclosure may be derived from the following description.

According to an aspect of the present disclosure, a print device that performs a print function is provided. The print device may be coupled to an auxiliary device that performs any one of functions including a skin guide, a hair guide, a multi-pass guide, and a camera accessory, be connected to the auxiliary device by recognizing a sensor attached to the auxiliary device or by recognizing the auxiliary device through wired or wireless communication with the auxiliary device, and change the function according to the type of the connected auxiliary device.

### BRIEF DESCRIPTION OF THE DRAWINGS

Embodiments of the inventive concept will be more clearly understood from the following detailed description taken in conjunction with the accompanying drawings in which:
FIG. 1 illustrates views of a print device that may be coupled to various auxiliary devices and an auxiliary device according to an embodiment of the present disclosure;
FIGS. 2 and 3 are views illustrating a process of recognizing an auxiliary device by using a recognition unit of the print device illustrated in FIG. 1;
FIG. 4 is a flowchart illustrating an operation process of the print device illustrated in FIG. 1;
FIGS. 5 to 20 are views and flowcharts illustrating a print device coupled to various auxiliary devices and an operation process thereof according to embodiments of the present disclosure; and
FIG. 21 is a flowchart illustrating a sequence of an operating method of a print device that may be coupled to various auxiliary devices, according to another embodiment of the present disclosure.

### DETAILED DESCRIPTION OF THE EMBODIMENTS

Hereafter, the present disclosure will be described in detail with reference to the accompanying drawings. However, the present disclosure may be implemented in many different forms and is not limited to the embodiments described herein. In addition, the accompanying drawings are only for easy understanding of the embodiments disclosed in the present specification, and the technical ideas disclosed in the present specification are not limited by the accompanying drawings. All terms, which include technical and scientific terms and are used herein, should be construed as meanings commonly understood by those skilled in the art in the technical field to which the present disclosure belongs. Terms defined in the dictionary should be construed as having additional meanings consistent with the related technical literature and currently disclosed content, and should not be construed in a very ideal or limited sense unless otherwise defined.

In order to clearly describe the present disclosure in the drawings, parts irrelevant to the descriptions are omitted, and a size, a shape, and a form of each component illustrated in the drawings may be variously modified. The same or similar reference numerals are assigned to the same or similar portions throughout the specification.

Suffixes "module" and "unit" for the components used in the following description are given or used interchangeably in consideration of ease of writing the specification, and do not have meanings or roles that are distinguished from each other by themselves. In addition, in describing the embodiments disclosed in the present specification, when it is determined that a detailed descriptions of related known technologies may obscure the gist of the embodiments disclosed in the present specification, the detailed descriptions are omitted.

Throughout the specification, when a portion is said to be "connected (coupled, in contact with, or combined)" with another portion, this includes not only a case where it is "directly connected (coupled, in contact with, or combined)" ", but also a case where there is another member therebetween. In addition, when a portion "includes (comprises or provides)" a certain component, this does not exclude other components, and means to "include (comprise or provide)" other components unless otherwise described.

Terms indicating ordinal numbers, such as first and second, used in the present specification are used only for the purpose of distinguishing one component from another component and do not limit the order or relationship of the components. For example, the first component of the present disclosure may be referred to as the second component, and similarly, the second element may also be referred to as the first component. As used herein, singular forms of expression should be construed to also include plural forms of expression, unless the contrary is clearly indicated.

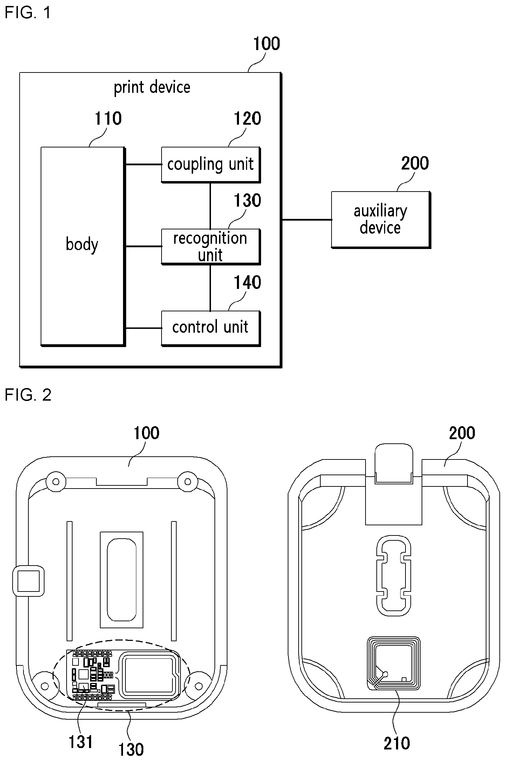

FIG. 1 illustrates views of a print device (hereinafter referred to as a "print device 100") that may be coupled to various auxiliary devices and an auxiliary device 200 according to an embodiment of the present disclosure.

Referring to FIG. 1, the print device 100 may be coupled to and connected to the auxiliary device 200. The print device 100 may perform a basic print function by using inkjet print technology. The print device 100 may recognize the auxiliary device 200. For example, the print device 100 may recognize the auxiliary device 200 by using various sensors (IR, photo interrupt, capacitive sensor, resistive sensor, and so on) or wired or wireless (radio frequency identification (RFID), a Universal Serial Bus (USB) cable, or so on) communication technology. According to the recognized auxiliary device 200, functions of the print device 100 and a terminal communicably connected to the print device 100 may be changed. A user may also select the auxiliary device 200 from an app of a user terminal that is communicably connected to the print device 100.

The print device 100 includes a body 110, a coupling unit 120, a recognition unit 130, and a control unit 140. The body 110 forms an appearance of the print device 100. The body 110 may be made of plastic or metal. The coupling unit 120 is formed on the body 110 to be physically coupled to the auxiliary device 200. The recognition unit 130 is formed on the body 110 to recognize the auxiliary device 200 and determine whether the coupling unit 120 is coupled to the auxiliary device 200. When the auxiliary device 200 is recognized, the recognition unit 130 may determine that the coupling unit 120 is coupled to the auxiliary device 200. When the recognition unit 130 recognizes that the auxiliary device 200 is not coupled to the coupling unit 120, the control unit 140 is set to perform a basic function. Also, when the recognition unit 130 recognizes that the auxiliary device 200 is coupled to the coupling unit 120, the control unit 140 is set to perform an individual function changed from the basic function according to the coupled auxiliary device 200. Although not illustrated in the drawing, the print device 100 may further include an ejection hole for ejecting ink and a storage box for storing ink. The ejection hole may be formed on in outer surface of the print device 100 to be exposed to the outside. The storage box may be placed inside the print device 100.

Although not illustrated in the drawing, the control unit 140 may include a memory and a processor to control functions of the print device 100. The memory may store at least one of information and data input to the print device 100, information and data required for functions performed by the processor, and data generated according to execution of the processor. For example, the memory may store an algorithm and program for printing ink, an algorithm or program for printing ink on skin or hair, and so on. The memory should be interpreted as a general term for a non-volatile memory device that maintains the stored information even when power is not supplied, and a volatile memory device that requires power to maintain the stored information. Also, the memory may perform a function of temporarily or permanently storing the data processed by the processor. The memory may include magnetic storage media or flash storage media in addition to the volatile memory device that requires power to maintain the stored information, but the scope of the present disclosure is not limited thereto. The processor is configured to execute a program stored in the memory. For example, the processor may execute a skin print program, a hair print program, and so on stored in the memory. The processor may include various types of devices that control and process data. The processor may refer to a data processing device that has a physically structured circuit to perform functions expressed by codes or instructions included in a program and is built in hardware. In one example, the processor may include a microprocessor, central processing unit (CPU), processor core, a multiprocessor, an application-specific integrated circuit (ASIC), a field programmable gate array (FPGA), or so on, but the scope of the present disclosure is not limited thereto.

The auxiliary device 200 may be formed of various auxiliary devices depending on the type of use to expand a function of the print device 100 and perform the function smoothly. For example, the auxiliary device 200 may be a skin guide, a hair guide, a makeup (color makeup) guide, a multi-pass guide, or a camera accessory. The auxiliary device 200 may be formed to guide the ink ejected by the print device 100 to regions, such as skin and hair.

Various methods may be applied to couple the print device 100 to the auxiliary device 200. In other words, the print device 100 may be connected to the auxiliary device 200 by using a fitting method, a screw coupling method, or so on. In one example, one side of the print device 100 may be formed as a bolt-shaped fastening structure, and one side of the auxiliary device 200 may be formed as a nut-shaped fastening structure. In this case, one side of the print device 100 and one side of the auxiliary device 200 may be coupled to each other to be in contact with each other. In this way, one side of the print device 100 and one side of the auxiliary device 200 may be formed in a structure that may be fastened to each other such that there is no space between the print device 100 and the auxiliary device 200.

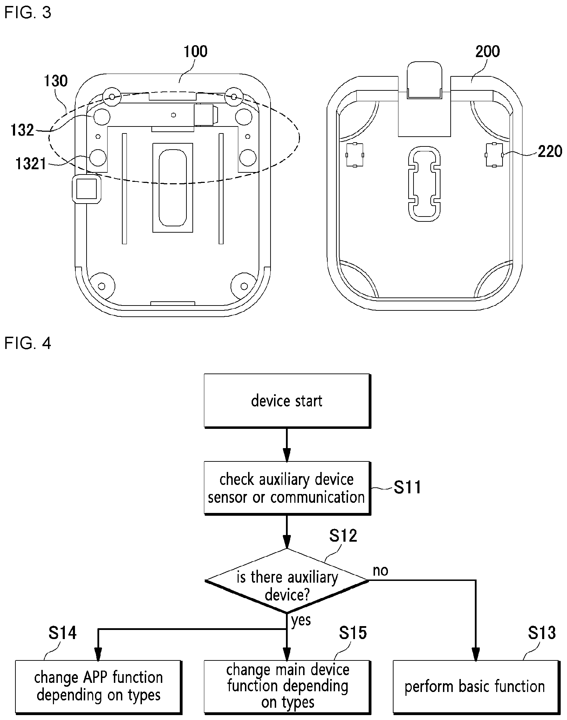

FIGS. 2 and 3 are views illustrating a process in which the recognition unit 130 of the print device 100of recognizes the auxiliary device 200.

Referring to FIG. 2, the recognition unit 130 may include a communication module 131. The recognition unit 130 may recognize the coupled auxiliary device 200 through wired or wireless communication by using the communication module 131. Due to this, the auxiliary device 200 may include a separate communication module 210. The communication module 131 transmits and receives information to and from an external device. Here, the external device may be an auxiliary device 200, an external server, or a terminal. The communication module 131 may include a device including hardware and software required to transmit and receive signals, such as control signals or data signals, through a wired or wireless connection to other network devices.

For example, a method by which the print device 100 recognizes the auxiliary device 200 by using communication technology is described below. The print device 100 may be connected to the auxiliary device 200 through proximity communication technology. The print device 100 may store information on the auxiliary device 200 in each tag by using proximity communication technology, such as RFID, and include a module or circuit that recognizes an auxiliary device therein. Thereafter, when the print device 100 may read tag information of the auxiliary device 200 when coupled to the auxiliary device 200 and distinguish each auxiliary device 200. Also, the print device 100 may be connected to the auxiliary device 200 through USB cable communication. When a USB cable connected to the auxiliary device 200 is directly connected to the print device 100, the print device 100 may read information of the auxiliary device 200. When power is required to be supplied to the auxiliary device 200, the power may be supplied from the print device 100. Unlike this, power may be supplied to the print device 100 and the auxiliary device 200 by a separate power supply. Also, the print device 100 may be connected to the auxiliary device 200 through RF communication. The auxiliary device 200 may include an RF communication module to perform wireless communication with the print device 100. Through this, the print device 100 may identify the type of auxiliary device 200 and perform an individual function according to the coupled auxiliary device.

Referring to FIG. 3, the recognition unit 130 may include a sensing module 132 including a sensor 1321 attached to the body 110. The recognition unit 130 may recognize a recognition target object 220 that is attached to the auxiliary device 200 and may be recognized by the sensor 1321, thereby recognizing the auxiliary device 200. In this case, the recognition unit 130 recognizes the auxiliary device 200 including the recognition object 220 recognized as another type as another auxiliary device 200. Here, the sensor 1321 may be a capacitive touch sensor, a hall sensor, or a photo sensor but is not limited thereto.

For example, a method by which the print device 100 recognizes the auxiliary device 200 by using a sensor is described below. The print device 100 may be automatically connected to the auxiliary device 200 through a connection method using a capacitive touch sensor. In this case, the print device 100 includes a circuit that recognizes a capacitive touch. The auxiliary device 200 includes a material that may be recognized by a touch sensor. The material is placed in a region of the auxiliary device 200 corresponding to a position of the touch sensor included in the recognition unit 130. Depending on the type of auxiliary devices 200, the number of metals may be adjusted in various ways. For example, an auxiliary device including one metal and an auxiliary device including two metals may be recognized as different auxiliary devices.

The print device 100 may be connected to the auxiliary device 200 through a connection method using a hall sensor. In this case, the print device 100 includes a hall sensor circuit. A magnet is provided in the auxiliary device 200. The magnet is placed in a region of the auxiliary device 200 corresponding to a position of the hall sensor. By adjusting the number of magnets depending on the type of auxiliary device 200, various auxiliary devices may be recognized and a function of each auxiliary device may be distinguished. For example, an auxiliary device including one magnet and an auxiliary device including two magnets may be recognized as different auxiliary devices.

The print device 100 may be connected to the auxiliary device 200 through a connection method using a photo sensor. In this case, the print device 100 includes a circuit to which a photo sensor is added. A structure that may cover the photo sensor is provided in the auxiliary device 200. The type of auxiliary device 200 may be changed depending on the number of structures to be covered.

FIG. 4 is a flowchart illustrating an operation process of the print device 100.

Referring to FIG. 4, first, the print device 100 checks sensors that sense an auxiliary device, a communication module, or so on (S11). Next, the print device 100 determines whether an auxiliary device is coupled or whether there is an auxiliary device (S12). When the auxiliary device 200 is not recognized because the auxiliary device 200 is not coupled to the print device 100, the print device 100 performs a basic function (S13). When the auxiliary device 200 is recognized because the auxiliary device 200 is coupled to the print device 100, the print device 100 may change a function of an application connected to the print device 100 depending on the type of the auxiliary device 200. The application may be executed by a terminal that is communicably connected to the print device 100. Also, when the auxiliary device 200 is recognized because the auxiliary device 200 is coupled to the print device 100, a function of the print device 100 is changed to an individual function depending on the type of the auxiliary device 200.

In one example, when a skin guide is recognized as being coupled to the coupling unit 120 by the recognition unit 130, the control unit 140 may set a skin print function according to a preset skin print algorithm corresponding to the skin guide as an individual function. In another example, when a hair guide is recognized as being coupled to the coupling unit 120 by the recognition unit 130, the control unit 140 may set a hair print function according to a preset hair print algorithm corresponding to the hair guide as an individual function. In another example, when a makeup guide is recognized as being coupled to the coupling unit 120 by the recognition unit 130, the control unit 140 may set a makeup print function according to a preset makeup print algorithm corresponding to the makeup guide as an individual function. In another example, when a multi-pass guide is recognized as being coupled to the coupling unit 120 by the recognition unit 130, the control unit 140 may set a multi-pass print function according to a preset multi-pass print algorithm corresponding to the multi-pass guide as an individual function. In another example, when a camera accessory is recognized as being coupled to the coupling unit 120 by the recognition unit 130, the control unit 140 may set a camera image print function according to a preset camera image print algorithm corresponding to the camera accessory as an individual function.

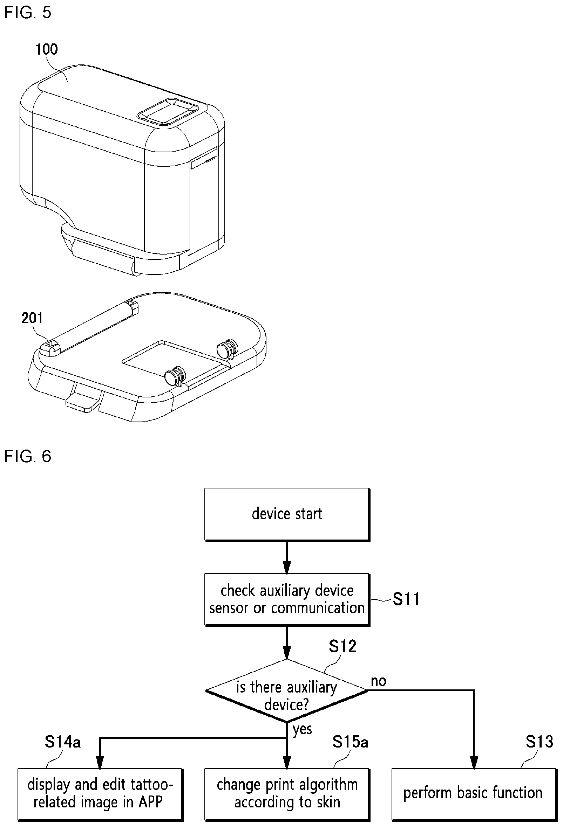

FIGS. 5 to 20 views and flowcharts illustrating functions of the print device 100 coupled to various auxiliary devices according to embodiments of the present disclosure. The functions of the print device 100 coupled to various auxiliary devices 201, 202, 203, 204, and 205 are described with reference to FIGS. 5 to 20.

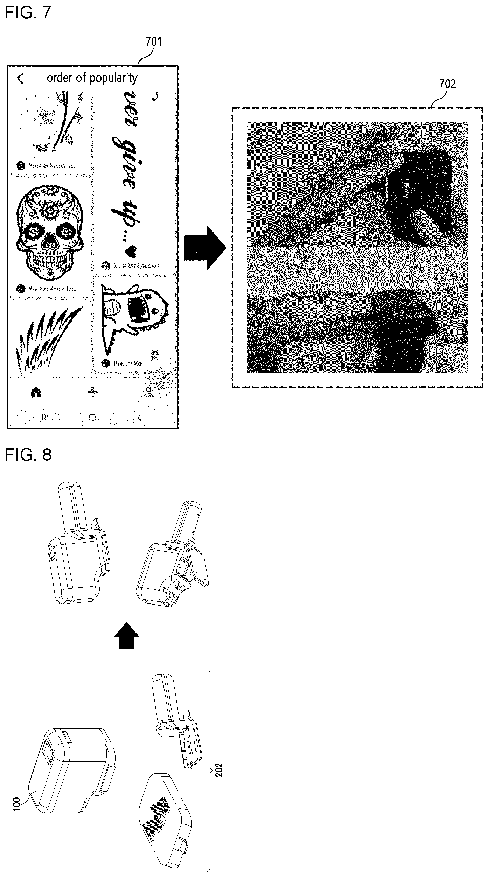

Referring to FIG. 5, the print device 100 may be coupled to a skin guide 201 which is one of the auxiliary devices 200. Referring to FIG. 6, an operation process of the print device 100 coupled to the skin guide 201 is similar to the operation process described with reference to FIG. 4. However, as the print device 100 is coupled to the skin guide 201, an interface and function of a terminal communicably connected to the print device 100 may be changed according to the skin guide 201 (S14a). Also, the function of the print device 100 may be changed to a skin print function according to a preset skin print algorithm in accordance with the skin guide 201 (S15a). Referring to FIG. 7, an interface displayed on a terminal may be configured as a tattoo image selection interface 701. When a user selects one of tattoo images included in a tattoo image selection interface 701 and places the print device 100 on or near the skin of oneself or another person, the print device 100 may print the selected tattoo image. The skin guide 201 may have a body that forms an appearance and includes a hollow region in the center. In this case, the ink ejected from an ejection hole of the print device 100 may be sprayed onto the hollow region of the body of the skin guide 201.

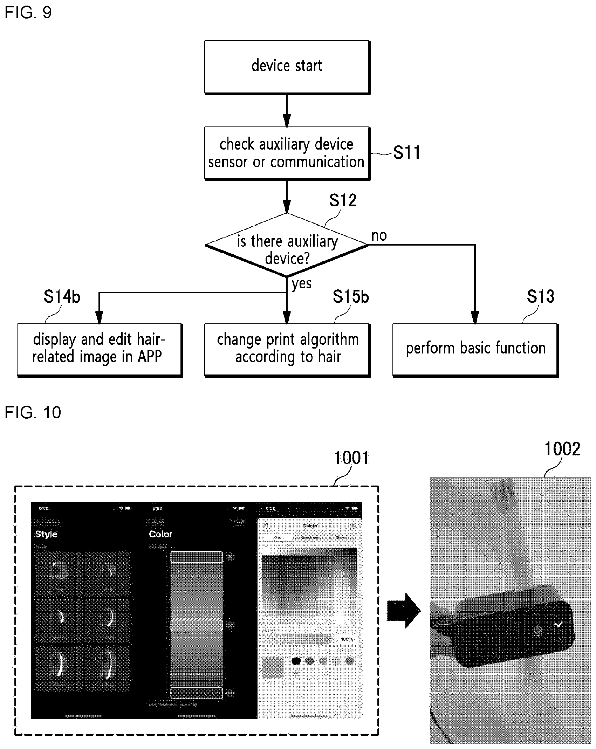

Referring to FIG. 8, the print device 100 may be coupled to the hair guide 202, which is one of the auxiliary devices 200. Referring to FIG. 9, an operation process of the print device 100 coupled to the hair guide 202 is similar to the operation process described with reference to FIG. 4. However, as the print device 100 is coupled to the hair guide 202, an interface and function of a terminal communicably connected to the print device 100 may be changed according to the hair guide 202 (S14b). Also, the function of the print device 100 may be changed to a hair print function according to a preset hair print algorithm in accordance with the hair guide 202 (S15b). Referring to FIG. 10, an interface displayed on a terminal may be configured as a hairstyle and color selection interface 1001. When you one of various hairstyles included in the hairstyle and color selection interface 1001 and one of various hair colors included therein are selected and the print device 100 is placed on or near a user' hair or another person's hair, the print device 100 may print the selected hair color according to the selected hairstyle (1002). Unlike the case of skin or paper, in the case of hair, a larger amount of ink needs to be sprayed than the existing printing amount to increase a color development effect. Also, in the case of hair, the brightness of hair changes depending on users, and accordingly, it is necessary to adjust the amount of ink printed according to light-colored hair and dark-colored hair. Therefore, when the hair guide 202 is mounted on the print device 100, the print device 100 may correct a print algorithm based on a color value designated as a hair color by a user or a color value automatically classified through a photo. The hair guide 202 may have a body that forms an appearance and includes a hollow region in the center. In this case, the ink ejected from the ejection hole of the print device 100 may be sprayed onto the hollow region of the body of the hair guide 202. In addition, the hair guide 202 may further include a comb-shaped guide unit that guides the ejected ink, and a handle unit that allows a user to easily operate the print device 100. The user may place the print device 100 near the hair by using the handle unit.

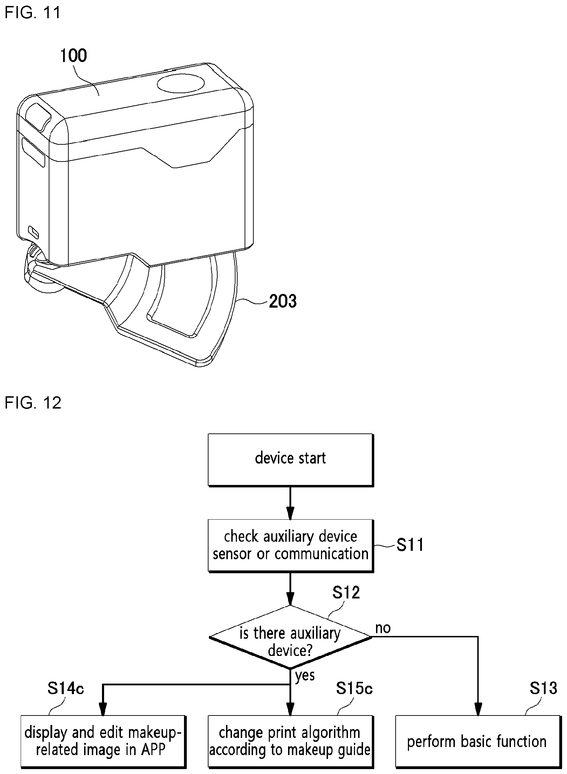

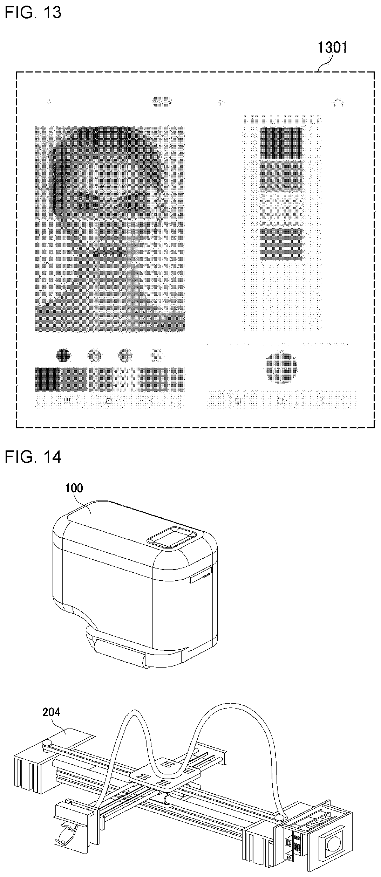

Referring to FIG. 11, the print device 100 may be coupled to the makeup guide 203 which is one of the auxiliary devices 200. Referring to FIG. 12, an operation process of the print device 100 coupled to the makeup guide 203 is similar to the operation process described with reference to FIG. 4. However, as the print device 100 is coupled to the makeup guide 203, the interface and function of the terminal communicated with the print device 100 may be changed according to the makeup guide 203 (S14c). Also, the function of the print device 100 may be changed to a makeup print function according to a preset makeup print algorithm in accordance with the makeup guide 203 (S15c). Referring to FIG. 13, an interface displayed on a terminal may be configured as a makeup selection interface 1301. When one of the various types of makeup information included in the makeup selection interface 1301 is selected and the print device 100 is placed on or near the face of oneself or another person, the print device 100 may print makeup according to the selected information (1002). The makeup guide 203 may have a body that forms an appearance and includes a hollow region in the center. In this case, the ink ejected from an ejection hole of the print device 100 may be sprayed onto the hollow region of the body of the makeup guide 203.

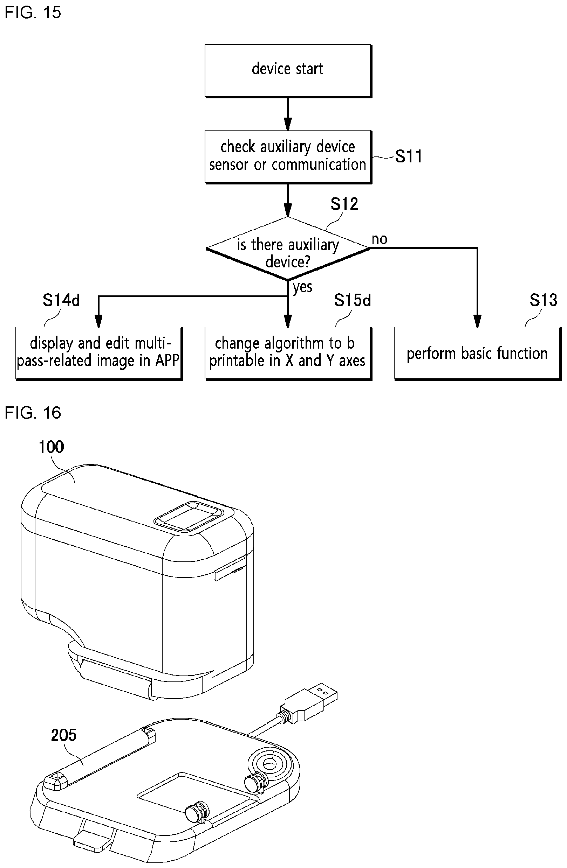

Referring to FIG. 14, the print device 100 may be coupled to a multi-pass guide 204 which is one of the auxiliary devices 200. Referring to FIG. 15, an operation process of the print device 100 coupled to the multi-pass guide 204 is similar to the operation process described with reference to FIG. 4. However, as the print device 100 is coupled to the multi-path guide 204, an interface and function of a terminal communicably connected to the print device 100 may be changed according to the multi-pass guide 204 (S14d). Also, the function of the print device 100 may be changed to a multi-pass print function according to a preset multi-pass print algorithm in accordance with the multi-pass guide 204 (S15d). According to the multi-pass guide 204 and the multi-pass print function, the print device 100 may be implemented to enable printing in x and y axes.

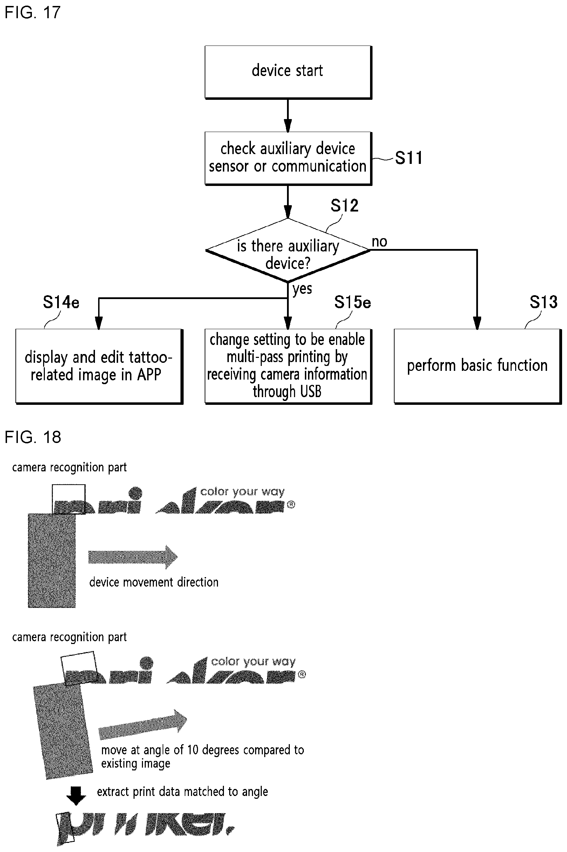

Referring to FIG. 16, the print device 100 may be coupled to the camera accessory 205 which is one of the auxiliary devices 200. Referring to FIG. 17, an operation process of the print device 100 coupled to the camera accessory 205 is similar to the operation process described with reference to FIG. 4. However, as the print device 100 is coupled to the camera accessory 205, an interface and function of a terminal communicably connected to the print device 100 may be changed according to the camera accessory 205 (S14e). Also, the function of the print device 100 may be changed to a camera image print function according to a preset camera image print algorithm in accordance with the camera accessory 205 (S15e). The camera accessory 205 may have a body that forms an appearance and includes a hollow region in the center. In this case, the ink ejected from an ejection hole of the print device 100 may be sprayed onto the hollow region of the body of the camera accessory 205.

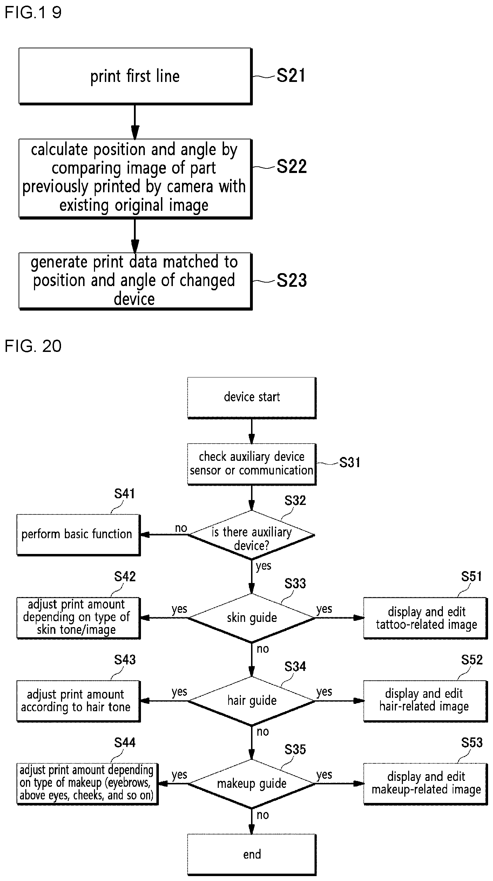

Referring to FIGS. 18 and 19, when the camera accessory 205 is coupled to the print device 100, the print device 100 may print a first line based on an original image (S21), calculate a position and angle by comparing an image of a part previously printed by a camera with the original image (S22), and continue printing according to a position and angle of the print device 100 which are changed according to the calculated position and angle (S23).

Referring to FIG. 20, examples of coupling, recognition, and an operation process of the skin guide 201, hair guide 202, makeup guide 203 and the print device 100 are described below. First, the print device 100 checks a sensor or communication module for sensing the auxiliary device 200 (S31). Next, the print device 100 recognizes the auxiliary device 200 and determines whether the print device 100 is coupled to the auxiliary device 200 (S32). When it is determined that the print device 100 is not coupled to the auxiliary device 200, the print device 100 performs a basic function (S41). When it is determined that the print device 100 is coupled to the auxiliary device 200, the print device 100 may determine whether the auxiliary device 200 is the skin guide 201 (S33), and when the auxiliary device 200 is not the skin guide 201, the print device 100 may determine whether the auxiliary device 200 is the hair guide 202, and, when the auxiliary device 200 is not the hair guide 202, the print device 100 may determine whether the auxiliary device 200 is the makeup guide 203 (S35).

When the coupled auxiliary device 200 is the skin guide 201, the print device 100 may adjust a print amount depending ona skin tone and the type of image printed on the skin (S42). Also, a tattoo-related image may be displayed or edited by using a terminal connected to the print device 100 (S51). When the coupled auxiliary device 200 is the hair guide 202, the print device 100 may adjust a print amount depending ona hair tone (S43). Also, a hair-related image may be displayed or edited by using a terminal connected to the print device 100 (S52). When the coupled auxiliary device 200 is the makeup guide 203, the print device 100 may adjust a print amount depending on the type of makeup (eyebrows, above eyes, cheeks, and so on) (S44). Also, a makeup-related image may be displayed or edited by using a terminal connected to the print device 100 (S53).

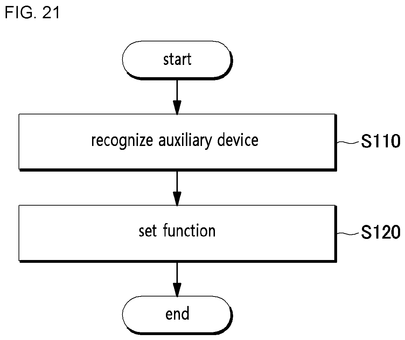

FIG. 21 is a flowchart illustrating a sequence of an operating method (hereinafter referred to as an "operating method of a print device") of a print device that may be coupled to various auxiliary devices, according to another embodiment of the present disclosure. An operating method of a print device to be described below may be performed by the print device (100 in FIG. 1) previously described with reference to FIGS. 1 to 20. Accordingly, the descriptions of the embodiments of the present disclosure previously described with reference to FIGS. 1 to 20 may be equally applied to embodiments to be described below, and redundant descriptions thereof are omitted below. Steps to be described below do not have to be performed in order, and a sequence of the steps may be set in various ways and may be performed almost simultaneously.

Referring to FIG. 21, an operating method of a print device is a method of operating a print device including a body that forms an appearance, a coupling unit formed on the body to be able to be physically coupled to an auxiliary device, a recognition unit formed on the body to recognize the auxiliary device and determine whether the coupling unit is coupled to the auxiliary device, and a control unit. Here, the body, the coupling unit, the recognition unit, and the control unit may be respectively the body (110 in FIG. 1), the coupling unit (120 in FIG. 1), the recognition unit (130 in FIG. 1), and the control unit (140 in FIG. 1) illustrated in FIG. 1.

The operating method of the print device includes an auxiliary device recognition step S110 and a function setting step S120. The auxiliary device recognition step S110 is a step in which the recognition unit recognizes the auxiliary device and determines whether the coupling unit is coupled to the auxiliary device. In the function setting step S120, when it is determined that the recognition unit is not coupled to the auxiliary device, the control unit sets a basic function to be performed, and when it is determined that the recognition unit is coupled to the auxiliary device, the control unit sets an individual function changed from the basic function according to the coupled auxiliary device to be performed.

In one example, the auxiliary device recognition step S110 may include a step in which the recognition unit recognizes the coupled auxiliary device through wired or wireless communication by using a communication module. In another example, the recognition unit may include a sensing module including a sensor attached to the body. In this case, the auxiliary device recognition step S110 may include a step in which the recognition unit is attached to the assistive device, an auxiliary device is recognized by recognizing a recognition target object that may be recognized by a sensor by using a sensing module, and auxiliary devices including the recognition object recognized as another type are recognized as different auxiliary devices.

The auxiliary device may be a skin guide, a hair guide, a makeup guide, a multi-pass guide, or a camera accessory. In this case, the function setting step S120 may include a step in which, when the skin guide is recognized as being coupled to the coupling unit by the recognition unit in the auxiliary device recognition step S110, the control unit sets a skin print function according to a preset skin print algorithm corresponding to the skin guide as an individual function, and when the hair guide is recognized as being coupled to the coupling unit by the recognition unit in the auxiliary device recognition step S110, the control unit sets a hair print function according to a preset hair print algorithm corresponding to the hair guide as an individual function, and when the makeup guide is recognized as being coupled to the coupling unit by the recognition unit in the auxiliary device recognition step S110, the control unit sets a makeup print function according to a preset makeup print algorithm corresponding to the makeup guide as an individual function, and when the multi-pass guide is recognized as being coupled to the coupling unit by the recognition unit in the auxiliary device recognition step S110, the control unit sets a multi-pass print function according to a preset multi-pass print algorithm corresponding to the multi-pass guide as an individual function, and when the camera accessory is recognized as being coupled to the coupling unit by the recognition unit in the auxiliary device recognition step S110, the control unit sets a camera image print function according to a preset camera image print algorithm corresponding to the camera accessory as an individual function.

Also, the function setting step S120 may include a step in which, when the recognition tool is recognized as being coupled to the coupling unit by the recognition unit, the control unit causes a terminal communicably connected to the print device to display an interface according to an individual function of the auxiliary device.

According to the present disclosure, a print device and auxiliary devices may be provided, the print device may perform most functions, such as printing, and auxiliary devices, each performing an individual function, may be used by being coupled to the print device. In this way, the print device according to the present disclosure may automatically recognize coupling to each of the different auxiliary devices by using a sensor or wired or wireless communication technology, and an operating method and a holding function may be changed according to the recognized auxiliary device.

Also, the present disclosure relates to a print device and may be used in a print industry, such as skin color print and hair color print, by using the print device.

The operating method of the print device according to the embodiments of the present disclosure described above may also be implemented in the form of a recording medium including instructions executable by a computer, such as a program module which is executed by a computer. A computer readable medium may be any available medium that may be accessed by a computer and includes both volatile and nonvolatile media, removable and non-removable media. Also, the computer readable medium may include a computer storage medium. A computer storage medium includes both volatile and nonvolatile media and removable and non-removable media implemented by any method or technology for storing information, such as computer readable commands, data structures, program modules or other data.

Those skilled in the technical field to which the present disclosure belongs will be able to understand that the present disclosure may be easily transformed into another specific form without changing technical idea or essential features based on the above description. Therefore, the embodiments described above should be understood as illustrative in all respects and not limiting. The scope of the present disclosure is indicated by the patent claims described below, and all changes or modified forms derived from the meaning and scope of the claims and their equivalent concepts should be construed as being included in the scope of the present disclosure. The scope of the present disclosure is indicated by the following claims rather than the detailed description above, and the meaning and scope of the claims and all changes or modifications derived from the equivalent concepts should be interpreted as being included in the scope of the present disclosure.

## Claims

1. A print device comprising:
a body forming an appearance;
a coupling unit formed in the body to enable physical coupling to an auxiliary device;
a recognition unit formed in the body to recognize the auxiliary device and determine whether the coupling unit is coupled to the auxiliary device; and
a control unit,
wherein, when the recognition unit recognizes that the auxiliary device is not coupled to the coupling unit, the control unit is configured to set the auxiliary device to perform a basic function, and when the recognition unit recognizes that the auxiliary device is coupled to the coupling unit, the control unit is configured to set the coupled auxiliary device to perform an individual function changed from the basic function.

2. The print device of claim 1, wherein
the auxiliary device is one of a skin guide, a hair guide, a makeup guide, a multi-pass guide, and a camera accessory.

3. The print device of claim 1, wherein
the recognition unit includes a communication module, and
the auxiliary device coupled through wired or wireless communication is recognized by using the communication module.

4. The print device of claim 1, wherein
the recognition unit includes a sensing module including a sensor attached to the body,
recognizes the auxiliary device by recognizing a recognition target object, which is attached to the auxiliary device and is recognizable by the sensor, by using the sensing module, and
recognizes the auxiliary device, which includes the recognition object recognized as another type, as another auxiliary device.

5. The print device of claim 4, wherein
the sensor is one of a capacitive touch sensor, a hall sensor, and a photo sensor.

6. The print device of claim 2, wherein,
when the recognition unit recognizes that the skin guide is coupled to the coupling unit, the control unit sets a skin print function according to a preset skin print algorithm corresponding to the skin guide as the individual function,
when the recognition unit recognizes that the skin guide is coupled to the coupling unit, the control unit 140 sets a hair print function according to a preset hair print algorithm corresponding to the hair guide as the individual function,
when the recognition unit recognizes that the skin guide is coupled to the coupling unit, the control unit sets a makeup print function according to a preset makeup print algorithm corresponding to the makeup guide as the individual function,
when the recognition unit recognizes that the skin guide is coupled to the coupling unit, the control unit sets a multi-pass print function according to a preset multi-pass print algorithm corresponding to the multi-pass guide as the individual function, and
when the recognition unit recognizes that the skin guide is coupled to the coupling unit, the control unit sets a camera image print function according to a preset camera image print algorithm corresponding to the camera accessory as the individual function.

7. The print device of claim 1, wherein,
when the recognition unit recognizes that the auxiliary device is coupled to the coupling unit,
the control unit causes a terminal communicably connected to the print device to display an interface according to the individual function of the auxiliary device.

8. An operating method of a print device including a body forming an appearance, a coupling unit formed in the body to enable physical coupling to an auxiliary device, a recognition unit formed in the body to recognize the auxiliary device and determine whether the coupling unit is coupled to the auxiliary device, and a control unit, the operating method comprising:
determining, by the recognition unit, whether the coupling unit is coupled to the auxiliary device by recognizing the auxiliary device; and
causing the control unit to set the auxiliary device to perform a basic function when the recognition unit recognizes that the auxiliary device is not coupled to the coupling unit, and causing the control unit to set the auxiliary device coupled to the auxiliary device to perform an individual function changed from the basic function when the recognition unit recognizes that the auxiliary device is coupled to the coupling unit.

9. The operating method of claim 8, wherein
the step of determining includes recognizing, by the recognition unit, the auxiliary device coupled through wired or wireless communication by using a communication module.

10. The operating method of claim 8, wherein
the recognition unit includes a sensing module including a sensor attached to the body, and
the step of determining includes recognizing, by the recognition unit, the auxiliary device by recognizing a recognition target object, which is attached to the auxiliary device and is recognizable by the sensor, by using the sensing module, and recognizing the auxiliary device, which includes the recognition target object recognized as another type, as another auxiliary device.

11. The operating method of claim 8, wherein
the auxiliary device is one of a skin guide, a hair guide, a makeup guide, a multi-pass guide, and a camera accessory, and
the step of causing of the control unit to set includes
causing the control unit to set a skin print function according to a preset skin print algorithm corresponding to the skin guide as the individual function when the recognition unit recognizes that the skin guide is coupled to the coupling unit in the step of determining,
causing the control unit to set a hair print function according to a preset hair print algorithm corresponding to the hair guide as the individual function when the recognition unit recognizes that the hair guide is coupled to the coupling unit in the step of determining,
causing the control unit to set a makeup print function according to a preset makeup print algorithm corresponding to the makeup guide as the individual function when the recognition unit recognizes that the makeup guide is coupled to the coupling unit in the step of determining,
causing the control unit to set a multi-pass print function according to a preset multi-pass print algorithm corresponding to the multi-pass guide as the individual function when the recognition unit recognizes that the multi-pass guide is coupled to the coupling unit in the step of determining, and
causing the control unit to set a camera image print function according to a preset camera image print algorithm corresponding to the camera accessory as the individual function when the recognition unit recognizes that the camera accessory is coupled to the coupling unit in the step of determining.

12. The operating method of claim 8, wherein
the step of causing of the control unit to set includes causing a terminal communicably connected to the print device to display an interface according to the individual function of the auxiliary device under control by the control unit when the recognition unit recognizes that the auxiliary device is coupled to the coupling unit.
